# EUROPÄISCHE PATENTANMELDUNG

(11) **EP 1 595 494 A1**
(43) Veröffentlichungstag der Anmeldung: **16.11.2005**
(21) Anmeldenummer: 05010491.8
(22) Anmeldetag: 13.05.2005
(51) Int. Cl.: A61B 5/00

(54) **Zahnärztliches System zum Untersuchen der optischen Eigenschaften von Zahngewebe mit optischer Untersuchungsvorrichtung und Abgleicheinrichtung**

(30) Priorität: 14.05.2004 DE 102004024164
(71) Anmelder: KALTENBACH & VOIGT GMBH & CO., 88400 Biberach (DE)
(72) Erfinder: Hack, Alexander, 88400 Biberach (DE); Erdmann, Sven, 89081 Ulm (DE); Heckenberger, Hans, 88433 Assmannshardt (DE)
(74) Vertreter: Schmidt-Evers, Jürgen

(57) **Zusammenfassung**

Eine Abgleicheinrichtung (50), die zur Durchführung eines Abgleichs einer Untersuchungsvorrichtung (1) mit Mitteln zum Erzeugen einer Anregungsstrahlung (A), welche auf einen zu untersuchenden Zahngewebebereich zu lenken ist, Erfassungsmitteln (40) und Auswertemitteln zum Erfassen und Bewerten einer von dem bestrahlten Zahngewebebereich als Antwort auf die Bestrahlung entstehenden Antwortstrahlung (F) sowie Übertragungsmitteln zum Übertragen der Anregungsstrahlung (A) sowie der Antwortstrahlung (F), welche mindestens eine Diagnosesonde (10) zum Ausrichten der Anregungsstrahlung (A) auf den zu untersuchenden Zahngewebebereich sowie zum Erfassen der Antwortstrahlung (F) umfassen, vorgesehen ist, weist ein Referenzelement (52) auf, welches zur Durchführung des Abgleichs der Untersuchungsvorrichtung (1) von dieser über die Diagnosesonde (10) zu bestrahlen ist. Erfindungsgemäße weist die Abgleicheinrichtung (50) eine Ablage (53) auf, über welche die Diagnosesonde (10) in einer definierten Position und/oder Orientierung bezüglich des Referenzelements (52) gehalten ist.

## Beschreibung

Die vorliegende Erfindung betrifft ein zahnärztliches System zum Untersuchen der optischen Eigenschaften von Zahngewebe, insbesondere zum Erkennen von Karies, Plaque, bakteriellem Befall, Konkrementen und Zahnstein, welches eine optische Untersuchungsvorrichtung sowie eine Abgleicheinrichtung zur Durchführung eines Abgleichs der Untersuchungsvorrichtung aufweist. Darüber hinaus betrifft die vorliegende Erfindung eine Abgleicheinrichtung zur Durchführung eines Abgleichs einer zahnärztlichen optischen Untersuchungsvorrichtung.

In der Dentaldiagnostik sind optische Untersuchungsvorrichtungen, mit deren Hilfe beispielsweise Karies, Plaque, bakterieller Befall, Konkremente oder Zahnstein erkannt werden kann, bereits seit längerem und in unterschiedlichen Varianten bekannt. Allen bekannten Vorrichtungen ist gemeinsam, dass ein zu untersuchender Zahngewebebereich zunächst mit einer Anregungsstrahlung bestrahlt wird, woraufhin von dem Zahn eine Antwortstrahlung abgegeben wird. Diese Antwortstrahlung kann sowohl die zurückreflektierte Strahlung gleicher Wellenlänge als auch eine Fluoreszenzstrahlung beinhalten. Die Antwortstrahlung wird wiederum erfaßt und einer Auswerteeinheit zugeführt, welche anhand des Spektrums der Antwortstrahlung ermittelt, ob eine der oben genannten Substanzen vorhanden ist oder nicht. Die bekannten optischen Diagnoseverfahren und Vorrichtungen unterscheiden sich dabei durch die verwendete(n) Wellenlänge(n) für die Anregungsstrahlung sowie durch die Auswertung der erfaßten Antwortstrahlung. Eine erste Möglichkeit besteht darin, zu untersuchen, ob an dem Zahn eine Fluoreszenzstrahlung als Reaktion auf die Anregungsstrahlung hin entstanden ist. Eine andere Möglichkeit besteht bei der sogenannten Reflexionsspektrometrie darin, zu untersuchen, welche Wellenlängen von der Zahnoberfläche in welcher Weise reflektiert werden.

Bekannte zahnärztliche Untersuchungsvorrichtungen zur Durchführung eines optischen Diagnoseverfahrens weisen grundsätzlich Mittel zum Erzeugen einer Anregungsstrahlung, welche auf den zu untersuchenden Zahngewebebereich zu lenken ist, Erfassungsmittel und Auswertemittel zum Erfassen und Bewerten einer von dem bestrahlen Zahngewebebereich als Antwort auf die Bestrahlung hin entstehenden Antwortstrahlung sowie Übertragungsmittel zum Übertragen der Anregungsstrahlung sowie der Antwortstrahlung auf. Üblicherweise beinhalten die Übertragungsmittel eine sog. Diagnosesonde, welche an dem zahnärztlichen Handstück, dass zur Durchführung der Untersuchung vorgesehen ist, angeordnet und dazu ausgebildet ist, die Anregungsstrahlung auf den zu untersuchenden Zahngewebebereich zu richten. Eine weitere Aufgabe der Diagnosesonde besteht darin, die Antwortstrahlung zu erfassen und zu den Erfassungs- und Auswertemitteln zu übertragen.

Obwohl zahnärztliche optische Untersuchungsvorrichtungen mit höchster Präzision gefertigt werden, kann im Laufe der Zeit eine Änderung in dem Messsignal entstehen, was auf eine Alterung der verschiedenen Bauteile sowie auf einen Verschleiß der Diagnosesonde zurückzuführen ist. Um daher reproduzierbare Ergebnisse zu erhalten, wird in regelmäßigen Abständen ein Abgleich der Untersuchungsvorrichtung durchgeführt. Ein derartiger Abgleich ist insbesondere auch dann wichtig, wenn über längere Zeit hinweg eine genaue Anzeige gefordert wird, was bspw. dann der Fall ist, wenn Überwachungsmaßnahmen über einen längeren Zeitraum von bspw. drei bis zwölf Monaten durchgeführt werden sollen. Ferner ist ein Abgleich auch deswegen erforderlich, da die Sonden regelmäßig ausgetauscht werden, um sie zu sterilisieren und zu desinfizieren.

Zur Durchführung des Abgleichs ist üblicherweise eine Abgleicheinrichtung vorgesehen, welche ein Referenzelement aufweist, das in spezifischer Weise auf die Bestrahlung mit der Anregungsstrahlung hin reagiert. Dieses Referenzelement besteht aus einem bestimmten Material, bspw. Keramik, welches bei Bestrahlung mit der Anregungsstrahlung eine bestimmte Antwortstrahlung erzeugt. Zur Durchführung des Abgleichs wird dann die Diagnosesonde der optischen Untersuchungsvorrichtung auf das Referenzelement gerichtet und eine Messung durchgeführt. Anhand des hierbei erhaltenen Messsignals wird dann ein Abgleich der Untersuchungsvorrichtung vorgenommen.

Bekannte Abgleicheinrichtungen zu Durchführung eines Abgleichs der Untersuchungsvorrichtungen weisen üblicherweise ein Referenzelement auf, auf welches die Diagnosesonde aufgesetzt wird. Der vorliegenden Erfindung liegt nunmehr die Aufgabe zugrunde, die bekannten Abgleicheinrichtungen derart weiter zu entwickeln, dass die Möglichkeiten zur Durchführung des Abgleichs weiter verbessert werden. Insbesondere soll sichergestellt werden, dass der Abgleich immer unter gleichbleibenden Bedingungen durchgeführt wird.

Die Aufgabe wird durch ein zahnärztliches System zum Untersuchen der optischen Eigenschaften von Zahngewebe gemäß Anspruch 1 sowie durch eine Abgleicheinrichtung gemäß Anspruch 9 gelöst.

Erfindungsgemäß ist vorgesehen, dass die Abgleicheinrichtung eine Ablage aufweist, über welche die Diagnosesonde der Untersuchungsvorrichtung in einer definierten Position und/oder Orientierung bezüglich des Referenzelements gehalten ist.

Gemäß der vorliegenden Erfindung ist somit die Abgleicheinrichtung derart ausgestaltet, dass bei der Durchführung des Abgleichs die Diagnosesonde grundsätzlich in der gleichen Position und Orientierung im Hinblick auf das Referenzelement angeordnet ist. Hierdurch wird eine hohe Reproduzierbarkeit der Messergebnisse erzielt und damit die Genauigkeit bei der Durchführung des Abgleichs verbessert.

Gemäß einem vorteilhaften Ausführungsbeispiel der vorliegenden Erfindung weist die Abgleicheinrichtung ein Gehäuse auf, in dem das Referenzelement gelagert ist, wobei die Ablage durch eine Ausnehmung in der Gehäusewandung gebildet ist, deren Form an die Kontur der Diagnosesonde angepasst ist. Insbesondere kann vorgesehen sein, dass die Ausnehmung in der Außenseite der Gehäusewandung der Abgleicheinrichtung gebildet ist, wobei das Gehäuse des weiteren dann eine Öffnung aufweist, durch welche sich die Diagnosesonde im abgelegenen Zustand erstreckt.

Zur Untersuchung der optischen Eigenschaften von Zahngewebe stehen üblicherweise unterschiedlich ausgestaltete Sonden zur Verfügung, die je nach dem Gebiet, in dem untersucht werden soll, ausgestaltet sind. Zur Untersuchung des sog. Approximalraums bzw. von Zahnzwischenräumen kommen beispielsweise vorwiegend Sonden zum Einsatz, die einen aus einem lichtleitenden Material bestehenden länglichen Lichtkeil aufweisen, an dessen vorderem Ende eine seitliche Auskopplung der Anregungsstrahlung erfolgt. Bei diesen Sonden ist es von besonderem Interesse, eine reproduzierbare Anordnung der Sonde während des Abgleichs zu erreichen, da andernfalls große Schwankung in dem Meßsignal auftreten könnten, welche einen Abgleich unter gleichbleibenden Bedingungen unmöglich machen.

Gemäß einem bevorzugten Ausführungsbeispiel der vorliegenden Erfindung weist deshalb die Ablage der Abgleicheinrichtung Mittel auf, welche eine gezielte Ausrichtung der Diagnosesonde in Richtung auf das Referenzelement hin ermöglichen. Beispielsweise könnte hierzu die Ausnehmung spezielle Markierungen oder Rastmittel aufweisen, welche im Zusammenwirken mit der Diagnosesonde eine korrekte Ausrichtung derselben sicherstellen.

Neben den zuvor angesprochenen Sonden, bei denen eine seitliche Lichtaus- und Lichteinkopplung erfolgt, kommen allerdings auch Sonden zur Untersuchung im Fissurenbereich zum Einsatz, bei denen das Licht mit Hilfe einer kalottenförmigen Spitze in Richtung der Längsachse der Sonde ausgekoppelt wird. Um auch für derartige Sonden eine möglichst reproduzierbare Anordnung im Hinblick auf das Referenzelement zu ermöglichen, ist gemäß einer vorteilhaften Weiterbildung vorgesehen, dass das Referenzelement eine rotationssymmetrische, insbesondere eine kalottenförmige Vertiefung an seiner Oberseite aufweist, in welche die Spitze der Sonde eingesetzt wird.

Die Verwendung dieser rotationssymmetrischen bzw. kalottenförmigen Vertiefung an der Oberseite des Referenzelements ist auch bei einem zweiten Ausführungsbeispiel der erfindungsgemäßen Abgleicheinrichtung von Vorteil, bei welcher die Sonde mit Hilfe eines Adapters definiert gehalten wird. Dieser Adapter wird auf das Gehäuse der Abgleicheinrichtung aufgesetzt und kann insbesondere mit diesem verrastbar sein. Dabei ist der Adapter derart ausgestaltet, dass die Spitze der Sonde in Anlage gegen die Oberfläche des Referenzelements gelangt und hierbei insbesondere auch in die Vertiefung eintaucht. Auf diese Weise wird eine besonders gut reproduzierbare Anordnung der Sonde erzielt, so dass die Qualität des Abgleichs sehr hoch ist.

Durch die erfindungsgemäße Lösung ist somit sichergestellt, dass bei unterschiedlichsten Sonden ein Abgleich der optischen Untersuchungsvorrichtung grundsätzlich unter gleichen Bedingungen durchgeführt werden kann, so dass auch über einen längeren Zeitraum hinweg vorgenommene Untersuchungen miteinander verglichen werden können.

Nachfolgend soll die vorliegende Erfindung anhand der beiliegenden Zeichnung näher erläutert werden. Es zeigen:
- Fig. 1: eine zahnärztliche optische Untersuchungsvorrichtung in Form eines Handstücks;
- Fig. 2a: die Ausgestaltung und Anordnung der wesentlichen Komponenten zur optischen Kariesdiagnose;
- Fig. 2b und 2c: vergrößerte Darstellungen von Fig. 2a;
- Fig. 3: eine zweite Variante der Untersuchungsvorrichtung mit einer weiteren Diagnosesonde;
- Fig. 4: die seitliche Ansicht eines ersten Ausführungsbeispiels einer Abgleicheinrichtung mit eingesetzter Diagnosesonde;
- Fig. 5: die Anordnung bestehend aus der Abgleicheinrichtung und der Diagnosesonde im seitlichen Schnitt;
- Fig. 6: eine perspektivische Darstellung der Ansicht von Fig. 5;
- Fig. 7: die Aufsicht der Anordnung von Fig. 6;
- Fig. 8: die Schnittdarstellung einer in die erfindungsgemäße Abgleicheinrichtung eingesetzten Diagnosesonde, die für Untersuchungen im Fissurenbereich vorgesehen ist,
- Fig. 9: ein zweites Ausführungsbeispiel einer Abgleicheinrichtung im seitlichen Schnitt und
- Fig. 10: die Abgleicheinrichtung von Fig. 9 in Aufsicht.

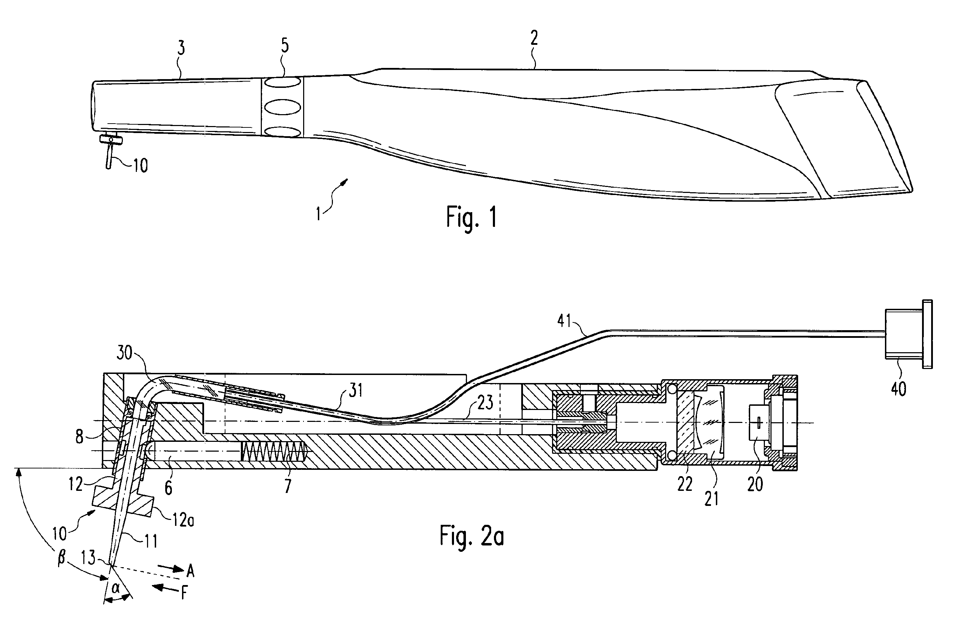

Fig. 1 zeigt die Aussenansicht einer zahnärztlichen Untersuchungsvorrichtung zum Untersuchen von Zahngewebe, insbesondere zum Erkennen von Karies, Plaque, bakteriellem Befall, Konkrementen oder Zahnstein. Die Vorrichtung ist im dargestellten Ausführungsbeispiel als Handstück 1 ausgestaltet, welches vollkommen unabhängig von weiteren externen Konsolen oder Auswerte- und Darstellungseinheiten verwendet werden kann. Der längliche Griffkörper 2 weist hierzu in seinem vorderen Kopfbereich 3 eine seitlich leicht schräg nach unten stehende Sonde 10 auf, welche zur Übermittlung einer Anregungsstrahlung auf den zu untersuchenden Zahngewebebereich sowie zur Übermittlung der von dem Zahn zurückgestrahlten Antwortstrahlung zu einer in dem Handstück 1 angeordneten Auswerteeinheit vorgesehen ist. Die genaue Ausgestaltung und Funktion der Diagnosesonde 10 wird später noch ausführlich erläutert.

Anzumerken ist, dass überwiegend auch optische Untersuchungsvorrichtungen zum Einsatz kommen, bei denen gewisse Bestandteile in externen Konsolen oder dergleichen angeordnet sind. Beispielsweise sind die Mittel zur Auswertung der Antwortstrahlung sowie zur Darstellung des Messergebnisses oftmals in Tischgeräten angeordnet, mit denen die Handstücke über Verbindungsschläuche verbunden sind. Die vorliegende Erfindung ist hiervon unabhängig und betrifft sowohl solche Untersuchungsvorrichtungen, die als vollkommen autonom arbeitende Handstücke ausgestaltet sind, als auch Vorrichtungen, bei denen ein Handstück mit weiteren Betriebsmitteln verbunden ist.

Im vorderen Bereich 3 des in Fig. 1 dargestellten Handstücks 1 ist ein Ringschalter vorgesehen, der zum Aktivieren der Anregungsstrahlungsquelle verwendet werden kann. Dieser Ringschalter kann Bestandteil einer abnehmbaren Hülse 2 sein, welche von den übrigen Komponenten des Handstücks 1 abgezogen werden kann, wodurch ein einfaches Reinigen und Sterilisieren derjenigen Teile ermöglicht wird, die mit dem Patienten in Kontakt gelangen.

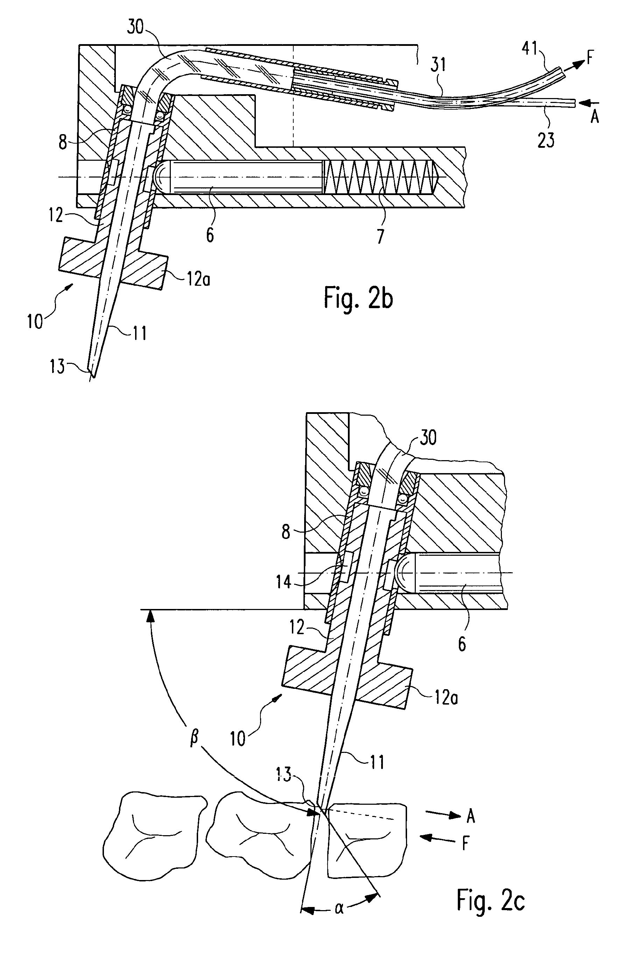

Die Fig. 2a bis 2c zeigen die Komponenten der Untersuchungsvorrichtung, die zum Erkennen der oben genannten Materialien an einem untersuchten Zahngewebebereich erforderlich sind. Wie bei den bekannten optischen Untersuchungsvorrichtungen erfolgt die Untersuchung dadurch, dass das zu untersuchende Zahngewebe einer Anregungsstrahlung ausgesetzt und die auf die Bestrahlung hin entstehende Antwortstrahlung erfasst und ausgewertet wird. Die wesentlichen Komponenten der Untersuchungsvorrichtung sind somit zum einen eine Lichtquelle zur Erzeugung der Anregungsstrahlung, Auswertemittel zum Auswerten der Antwortstrahlung sowie Übertragungsmittel zum Übertragen der Antwortstrahlung zu dem zu untersuchenden Bereich sowie zum Übertragen der Antwortstrahlung zu den Auswertemitteln hin.

Bei dem dargestellten Handstück 1 wird die Lichtquelle zur Erzeugung der Anregungsstrahlung durch eine Laserdiode 20 gebildet, welche ein nahezu monochromatisches Licht erzeugt. Insbesondere kann die Anregungsstrahlung im Bereich zwischen 600 nm und 670 nm, vorzugsweise bei ca. 655 nm liegen, da bei einer derartigen Wellenlänge der bestmögliche Kompromiß zwischen der Ausgangsleistung der Laserdiode 20 und dem spektralen Unterschied zwischen der Anregungsstrahlung und der von der Zahnoberfläche zurückgeworfenen Antwortstrahlung erzielbar ist. Anzumerken ist, dass die Funktion einer optischen Untersuchungsvorrichtung hier am Beispiel einer Fluoreszenzdiagnose erläutert wird, bei der die an der Zahnoberfläche als Reaktion auf die Bestrahlung hin entstehende Fluoreszenzstrahlung bewertet wird. Alternativ dazu bestünde allerdings auch die Möglichkeit, andere Wellenlängen für die Anregungs- und/oder Antwortstrahlung zu verwenden oder im Rahmen einer sog. Reflexionsspektrometrie zur untersuchen, welche Wellenlängen von der Zahnoberfläche in welcher Weise reflektiert werden.

Mit Hilfe einer der Laserdiode 20 vorgeschalteten Linse 21 sowie einem optischen Filter 22, der das von der Laserdiode 20 abgegebene Licht nochmals auf den gewünschten Wellenlängenbereich eingrenzt, wird dann eine Anregungsstrahlung A erzeugt und in einen ersten Lichtleiter 23 eingekoppelt. Bei diesem Lichtleiter 23 kann es sich um eine einzelne Lichtfaser mit einem Durchmesser von ca. 0,5 mm handeln, es bestünde allerdings auch die Möglichkeit, den Lichtleiter 23 aus einer Vielzahl von einzelnen Lichtleiterfasern zu bilden. An seinem vorderen Ende grenzt der Lichtleiter 23 an einen gekrümmten Faserstab 30 aus einem ebenfalls lichtleitenden Material an, durch den die Anregungsstrahlung umgelenkt und in die Stirnseite der Diagnosesonde 10 eingekoppelt wird.

Im dargestellten Ausführungsbeispiel handelt es sich um eine Sonde 10 zur Untersuchung von Zahnzwischenräumen. Wesentliches Element der Sonde 10 ist ein länglicher Lichtkeil 11 aus einem transparenten Material, an dessen unterem Ende die Anregungsstrahlung A ausgekoppelt und auf den zu untersuchenden Zahnbereich gerichtet wird. Als Material für den Lichtkeil 11 kann beispielsweise Kunststoff oder Saphir verwendet werden, wobei Kunststoff hinsichtlich der geringeren Bruchgefahr und Herstellungskosten vorteilhaft ist, allerdings Nachteile im Hinblick auf Abnutzungserscheinungen und die daraus resultierende Lebensdauer aufweist.

Wie insbesondere der Darstellung in Fig. 2c entnommen werden kann, soll das Licht seitlich zur Längsachse aus dem Lichtkeil 11 ausgekoppelt werden, um eine Untersuchung der Zahnzwischenräume zu ermöglichen. Hierzu ist das vordere Ende des aus einem transparenten Material bestehenden Lichtkeils 11 mit einer Schräge 13 versehen, die mit der Längsachse des Lichtkeils einen Winkel α von ca. 40 bis 45° einschließt. Das von oben einfallende Licht wird dann an dieser Schräge 13 totalreflektiert und seitlich aus dem Lichtkeil 11 ausgekoppelt. Zusätzlich oder alternativ könnte die Schräge 13 auch verspiegelt sein, um die Umlenkung des Licht zu erzielen.

Die Rückübermittlung der auf die Bestrahlung hin an der Zahnoberfläche entstehenden Antwortstrahlung F erfolgt in ähnlicher Weise in umgekehrter Richtung. Zunächst fällt die Antwortstrahlung seitlich in den Lichtkeil 11 ein und wird wiederum an der Schräge 13 reflektiert und somit zur Stirnseite der Sonde 10 gelenkt und in den Faserstab 30 eingekoppelt. Von dem Ende des Faserstabs 30, der wiederum vorzugsweise aus einer Vielzahl von Einzelfasern mit einem Durchmesser von 0,1 mm besteht und insgesamt einen Durchmesser von ca. 1,4 mm aufweist, wird dann die Antwortstrahlung in ein Lichtfaserbündel 31 eingekoppelt, welches zum einen aus der bzw. den Anregungsstrahlungsfaser(n) 23 für die Anregungsstrahlung A und zum anderen aus einer Detektionsfaser 41 zur Übermittlung der Antwortstrahlung F besteht.

Über die Detektionsfaser 41, die vorzugsweise einen Durchmesser von 0,25 mm aufweist, erfolgt dann die Weiterleitung zu einer Erfassungseinrichtung 40, deren Aufgabe es ist, die von der Zahnoberfläche zurückgestrahlte Antwortstrahlung zu erfassen, zu analysieren und auf Basis des Meßergebnisses zu beurteilen, ob eine der eingangs genannten fluoreszierenden Substanzen an der untersuchten Zahnoberfläche vorhanden ist oder nicht.

Zu der Diagnosesonde 10 ist anzumerken, dass diese in dem Kopfbereich 3 des Handstücks 1 um 360° drehbar gelagert ist, so dass das Handstück 1 sehr flexibel an die zu untersuchenden Zähne herangeführt werden kann. Die Sonde 10 ist dabei mit dem Kopfbereich 3 des Handstücks 3 verrastet und kann in sehr einfacher Weise - z.B. zu Reinigungszwecken oder zum Ersetzen durch eine andere Sonde - entnommen werden. Hierzu sind in dem Kopfbereich 3 des Handstücks 1 zunächst eine zylinderförmige Führung 8 für die Sonde 10 sowie ein Raststift 6 vorgesehen, der mit Hilfe einer Feder 7 gegen die Sonde 10 drückt und diese damit in der eingesetzten Position innerhalb des Führung 8 hält. Der vordere halbkugelförmige Endbereich des Raststifts 6 greift dabei in eine umlaufende Ausnehmung 14 auf die Kante eines Halters 12 für den Lichtkeil 11 ein, so dass die Sonde 10 sicher innerhalb des Handstücks 1 aber zugleich drehbar gelagert ist. Eine Drehung der Sonde 12 von Hand wird dabei durch einen scheiben- oder ringförmigen Aufsatz 12a an dem Halter 12 erleichtert, der von einem Benutzer des Handstücks 1 mit den Fingern leicht gegriffen und verdreht werden kann. Der Halter 12 selbst weist eine längliche Bohrung auf, in welche der Lichtkeils 11 eingesetzt ist, wobei die Möglichkeit besteht, den Lichtkeil 11 auszuwechseln.

Anzumerken ist ferner, dass die Diagnosesonde 10 gegenüber der Handstücklängsachse nicht in einem rechten Winkel, sondern vorzugsweise leicht schräg in einem Winkel β von ca. 80° gehalten wird. Es hat sich herausgestellt, dass hierdurch eine besonders ergonomisch günstige Handhabung der erfindungsgemäßen Untersuchungsvorrichtung erzielt wird.

Die lösbare Halterung der Sonde 10 bringt zum einen den Vorteil mit sich, dass die Sonde 10 nach jeder Untersuchung entfernt und getrennt von den übrigen Bestandteilen des Handstücks 1 gereinigt und desinfiziert werden kann. Zum anderen besteht allerdings auch der Vorteil, dass die Sonde 10 leicht ausgetauscht und durch eine anders gestaltete Sonde ersetzt werden kann. Es besteht hierdurch die Möglichkeit, unterschiedlich geformte Sonden bereitzustellen, die je nach Ort bzw. Oberflächengestaltung der zu untersuchenden Zahnstelle ausgestaltet sein können.

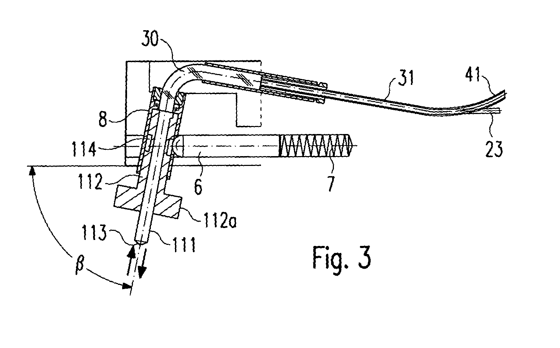

Fig. 3 zeigt eine weitere Sonde, die üblicherweise bei einer optischen Untersuchungsvorrichtung zum Einsatz kommen kann. Bei der in Fig. 3 dargestellten Sonde 110 handelt es sich um eine Sonde, die zu Untersuchungen im Fissurenbereich - also zur Untersuchung der Kauflächen von Zähnen oder von Glattflächen bzw. Zahnaußenflächen - vorgesehen ist. Im Gegensatz zu der in den vorherigen Figuren dargestellten Sonde 10 zur Untersuchung des Approximalraums bzw. Zahnzwischenraums weist die Sonde 110 keine Abschrägung an der Sondenspitze sondern stattdessen eine kegelstumpfförmige Lichtaustrittspitze 113 auf. Die Sonde 110 besteht wiederum aus einem länglichen Lichtkeil 111 aus einem lichtleitenden Material mit der Lichtaustrittsspitze 113, wobei der Lichtkeil 111 durch einen Halter 112 mit einem scheibenförmigen Aufsatz 112a gehalten ist, welcher im hinteren Bereich die umlaufende Ausnehmung 114 aufweist, über die eine Verrastung mit dem Handstück 1 erzielt wird.

Die in den Fig. 2a bis 2c bzw. 3 dargestellten Sonden 10 und 110 müssen bei einer Benutzung der Untersuchungsvorrichtung regelmäßig gewechselt werden. Der Grund hierfür kann sein, dass andere Bereiche eines Zahns untersucht werden sollen und dementsprechend die Verwendung einer anders gestalteten Sonde erforderlich ist. Darüber hinaus ist selbstverständlich auch ein Wechsel der Sonden aus hygienischen Gründen erforderlich, um die Sonden nach Gebrauch zu reinigen und/oder zu sterilisieren.

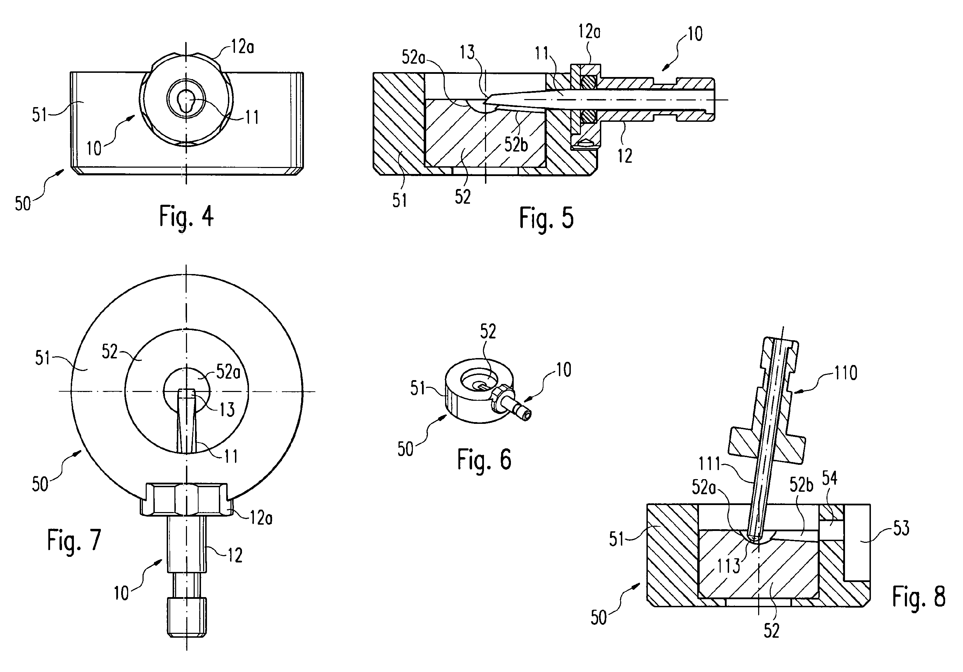

Da durch einen Wechsel der Sonden sich die Verhältnisse bei der Übertragung der Anregungs- und Antwortstrahlung allerdings verändern und darüber hinaus auch Abnutzungserscheinungen im Laufe der Zeit auftreten können, ist es erforderlich, die optische Untersuchungsvorrichtung regelmäßig abzugleichen. Eine Abgleicheinrichtung, welche in besonders effektiver Weise eine reproduzierbare Anordnung der Sonden und damit einen sehr genauen Abgleich ermöglicht, soll nunmehr anhand der Fig. 4 bis 8 erläutert werden.

Die in den Fig. 4 bis 8 dargestellte erfindungsgemäße Abgleicheinrichtung 50 besteht aus einem topfförmigen Gehäuse 51 mit einer zylinderförmigen Wand, welches zur Lagerung eines Referenzelements 52 vorgesehen ist. Bei dem Referenzelement 52 handelt es sich um ein Keramikelement oder um ein anderes Material, welches in spezifischer Weise auf die Anregungsstrahlung der Untersuchungsvorrichtung hin reagiert. Anstelle eine Zylinderform könnte für die Abgleicheinrichtung 50 auch jede andere geeignete Form gewählt werden.

Der Abgleich der Untersuchungsvorrichtung ist dadurch durchzuführen, dass die Sonde auf das Referenzelement 52 hin ausgerichtet und eine Messung in üblicher Weise durchgeführt wird, wobei anhand des Messergebnisses dann ein Abgleich der Untersuchungsvorrichtung erfolgt.

Um reproduzierbare Ergebnisse zu erhalten und einen genauen Abgleich der Untersuchungsvorrichtung zu ermöglichen, ist es erforderlich, dass die Sonde im Hinblick auf das Referenzelement 52 grundsätzlich in gleicher Weise angeordnet wird. Erfindungsgemäß sind hierzu an der Abgleicheinrichtung 50 Maßnahmen vorgesehen, die dieses sicher stellen.

Wie den Darstellungen in den Fig. 4 bis 7 entnommen werden kann, ist in der Gehäusewandung eine Ausnehmung 53 vorgesehen, in welcher die Sonde 10 zur Untersuchung des Approximalraums bzw. von Zahnzwischenräumen gelagert werden kann. Die Kontur dieser Ausnehmung 53 entspricht dabei zumindest teilweise den Abmessungen des ringförmigen Aufsatzes 12a, so dass eine definierte Lagerung der Sonde 10 im Hinblick auf das Referenzelement 52 gewährleistet ist. In der Gehäusewandung ist darüber hinaus eine Durchgangsöffnung bzw. Bohrung 54 vorgesehen, durch die sich der längliche Lichtkeil 11 erstreckt, wobei die Abmessungen des Gehäuses 51 insgesamt derart gewählt werden, dass die Sondenspitze des Lichtkeils 11 über dem Zentrum des Referenzelements 52 gelagert ist.

An der Ausnehmung 53 bzw. der Gehäusewandung der Abgleicheinrichtung 50 können Maßnahmen vorgesehen sein, die sicherstellen, dass die Sonde 10 im Hinblick auf das Referenzelement 52 hin richtig orientiert ist. Zur Durchführung des Abgleichs ist - wie insbesondere der Darstellung in Fig. 5 entnommen werden kann - erforderlich, dass die für die seitliche Auskopplung der Anregungsstrahlung erforderliche Schräge 13 derart ausgerichtet ist, dass das Licht senkrecht auf das Referenzelement 52 gerichtet wird. Entsprechende Maßnahmen können bspw. durch - nicht dargestellte - Rastelemente oder Markierungen in der Gehäusewandung gebildet sein.

Wie den Darstellungen entnommen werden kann, ist aufgrund dieser Maßnahmen also sichergestellt, dass die Diagnosesonde 10 bei jedem Abgleich in gleicher Weise im Hinblick auf das Referenzelement 52 angeordnet und ausgerichtet ist, so dass bei der Durchführung des Abgleichs immer die gleichen Bedingungen vorliegen und dementsprechend eine hohe Reproduzierbarkeit erzielt wird.

Um sicherzustellen, dass die Sonde 10, genaugenommen die Sondenspitze 13, aus der die Anregungsstrahlung ausgekoppelt wird, in unmittelbarer Nähe zu dem Referenzkörper 52 angeordnet ist, ist eine sich vom Zentrum zu der Öffnung 54 in der Gehäusewandung vorgesehene Ausnehmung 52b vorgesehen, innerhalb deren der längliche Lichtkeil 11 der Sonde 10 gelagert ist.

Darüber hinaus weist das Referenzelement 52 auch eine kalottenförmige Vertiefung 52a im Zentrum der Oberfläche auf, welche gemäß der Darstellung in Fig. 8 dazu vorgesehen ist, die Sondenspitze der Sonde 110, die zur Untersuchung im Fissurenbereich vorgesehen ist, aufzunehmen. Im Gegensatz zu der Sonde 10 zur Untersuchung des Approximalraums ist bei dieser Sonde 110 für den Fissurenbereich eine bestimmte Orientierung der Sondenspitze bzw. des Lichtkeils 111 weniger wichtig, da bei dieser Sonde 110 die Anregungsstrahlung grundsätzlich in Richtung der Längsachse austritt. Es ist allerdings erforderlich, dass die kalottenförmige Sondenspitze 113 möglichst genau auf dem Referenzelement 52 zur Auflage kommt.

Durch die kalottenförmige Ausnehmung 52a ist nunmehr sichergestellt, dass die Sondenspitze 113 grundsätzlich unter gleichen Bedingungen im Hinblick auf die Oberfläche des Referenzelements 52 angeordnet ist. Somit wird auch für diesen Sondentyp ein genauer Abgleich ermöglicht. Auch der Abgleich einer sog. Parosonde, die zur Untersuchung von Zahnfleischtaschen und insbesondere zum Lokalisieren von subgingivalen Konkrementen an Zahnwurzeln vorgesehen ist, kann mit Hilfe der kalottenförmige Ausnehmung 52a durchgeführt werden. Eine derartige Sonde ist am äußersten Ende ihrer Spitze gerade abgeflacht, wodurch ein sehr schlanker Lichtaustritt mit einem kleinen Durchmesser erzielt wird, so dass die Anregungsstrahlung sehr konzentriert auf eine zu untersuchende Stelle gerichtet werden kann. Auch bei dieser Sonde erfolgt der Lichtaus- und Lichteintritt in Richtung der Längsachse, so dass durch die Gestalt der Ausnehmung 52a wiederum ein Abgleich unter gleichbleibenden Bedingungen ermöglicht wird.

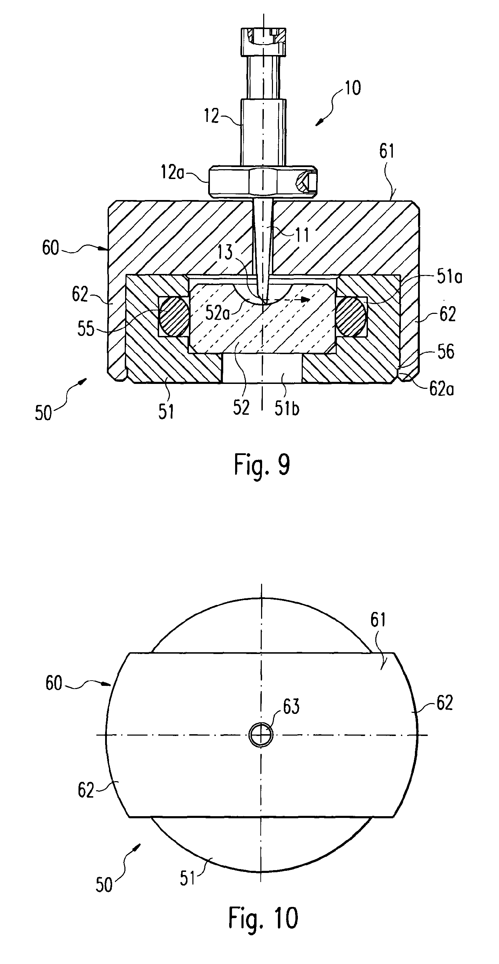

Eine Variante der in den Fig. 4 bis 8 dargestellten Abgleicheinrichtung ist in den Fig. 9 und 10 dargestellt. Hierbei sind gleiche Elemente der Abgleicheinrichtung mit den gleichen Bezugszeichen versehen. Wiederum besteht die Abgleicheinrichtung 50 zunächst aus einem Gehäuse 51, in dem ein Referenzelement 52 gelagert ist. Im vorliegenden Fall wird das Referenzelement 52 mit Hilfe eines flexiblen O-Rings 55 fixiert, der innerhalb einer ringfömigen Gehäuseausnehmung 51a angeordnet ist. Über eine an der Unterseite des Gehäuses 51 vorgesehene Öffnung 51b kann dann das Referenzelement 52 zur Oberseite hin ausgestoßen werden, falls ein Austausch des Referenzelements 52 erforderlich ist. Diese Art der auswechselbaren Anordnung des Referenzelements 52 in dem Gehäuse 51 könnte selbstverständlich auch bei dem vorherigen Ausführungsbeispiel der erfindungsgemäßen Abgleicheinrichtung vorgesehen sein.

Im Gegensatz zu dem Ausführungsbeispiel gemäß den Fig. 4 bis 8 erfolgt eine definierte Anordnung der Sonde - insbesondere der Sonde 10 zur Untersuchung des Approximalraums - nicht über eine in der Seitenwand des Gehäuses 51 vorgesehene Ausnehmung, sondern statt dessen mit Hilfe eines klammerförmigen Adapters 60, der von der Oberseite her auf das zylinderförmige Gehäuse 51 der Abgleicheinrichtung 50 aufgesetzt werden kann. Entsprechend der Darstellung besteht der Adapter 60 aus einer etwa rechteckigen, horizontal ausgerichteten Grundfläche 61, die an ihren Stirnseite zwei nach unten weisende Klemmarme 62 aufweist, welche beim Aufsetzen des Adapters 60 auf das Gehäuse 51 über die Seitenwand des Gehäuses 51 geschoben werden. Über an den Unterseiten der Arme 62 vorgesehene Rastvorsprünge 62a, welche mit entsprechenden Vorsprüngen 56 des Gehäuses 51 zusammenwirken, ist eine sichere Halterung des Adapters 60 gewährleistet.

Im Zentrum der Grundfläche 61 des Adapters 60 ist eine Durchgangsbohrung 63 vorgesehen, durch welche der Lichtkeil 11 der Sonde 10 geschoben werden kann. Die Dicke der Grundplatte 61 ist dabei derart bemessen, daß, wenn der ringförmige Aufsatz 12a des Halters 12 der Sonde 10 auf der Oberseite der Grundfläche 61 aufliegt, die Sondenspitze 13 ebenfalls in Anlage gegen das Referenzelement kommt. Genau genommen, taucht die Sondenspitze 113 in die rotationssymmetrische Ausnehmung 52a des Referenzelements 52 ein und kommt gegen die Bodenseite der Ausnehmung 52a zur Anlage.

Auch auf diese Weise ist sichergestellt, daß die Sondenspitze 113 in einer gewünschten Weise auf das Referenzelement 52 ausgerichtet ist. Da die Sonde 10 grundsätzlich auf Anschlag angeordnet ist, wird eine hohe Wiederholgenauigkeit erzielt, was für die Durchführung des Abgleichs von besonderer Relevanz ist. Ferner ist in diesem Fall keine axiale Ausrichtung der Sonde 10 erforderlich, da aufgrund der rotationssymmetrischen Ausgestaltung der Ausnehmung 52a immer gleiche Bedingungen vorliegen. In gleicher Weise, also mit Hilfe des Adapters, könnte im übrigen auch die in Fig. 8 dargestellte Sonde 110 angeordnet werden, wobei allerdings die Verwendung des Adapters 60 bei diesem Sondentyp nicht zwingend erforderlich ist.

Der Vorteil dieser zweiten Ausführungsform der erfindungsgemäßen Abgleicheinrichtung 50 besteht darin, daß aufgrund der Anordnung in Kontakt mit der Oberfläche des Referenzelements 52 eine besonders gute Reproduzierbarkeit erzielt wird. Bei dem Ausführungsbeispiel gemäß den Fig. 4 bis 8 hingegen schwebt die Approximalsonde über der Ausnehmung 52a des Referenzelements 52. Wenn nun anstelle von Saphirsonden Kunststoffsonden Verwendung finden, könnte durch eventuelle Ungenauigkeiten die Kunststoffsonde beim Einstecken in die Vorrichtung verbogen werden. Hierdurch kann es zu geringfügigen Abstandsänderungen kommen, die zu deutlichen Veränderungen in den Signalwerten führen, was letztendlich die Genauigkeit bei der Durchführung des Abgleichs reduziert. Diese Nachteile werden bei der Ausführungsform gemäß den Fig. 9 und 10 vermieden.

Den vorherigen Schilderungen kann entnommen werden, dass durch spezielle Maßnahmen an der Abgleicheinrichtung sichergestellt ist, dass die Untersuchungssonden der optischen Untersuchungsvorrichtung grundsätzlich in gleicher Weise im Hinblick auf das Referenzelement bzw. dessen Oberfläche angeordnet und ausgerichtet werden. Die unerlässlichen Abgleiche der Untersuchungsvorrichtung können somit grundsätzlich unter gleichen Bedingungen durchgeführt werden, wodurch besonders genaue Ergebnisse erzielt werden. Hierdurch ist sichergestellt, dass auch über einen längeren Zeitraum hinweg vorgenommene Messungen miteinander vergleichbar sind.

## Patentansprüche

1. Zahnärztliches System zum Untersuchen der optischen Eigenschaften von Zahngewebe, insbesondere zum Erkennen von Karies, Plaque, bakteriellem Befall, Konkrementen und Zahnstein, aufweisend
a) eine optische Untersuchungsvorrichtung (1) mit
- Mitteln zum Erzeugen einer Anregungsstrahlung (A), welche auf einen zu untersuchenden Zahngewebebereich zu lenken ist,
- Erfassungsmitteln (40) und Auswertemitteln zum Erfassen und Bewerten einer von dem bestrahlten Zahngewebebereich als Antwort auf die Bestrahlung entstehenden Antwortstrahlung (F) sowie
- Übertragungsmitteln zum Übertragen der Anregungsstrahlung (A) sowie der Antwortstrahlung (F), welche mindestens eine Diagnosesonde (10) zum Ausrichten der Anregungsstrahlung (A) auf den zu untersuchenden Zahngewebebereich sowie zum Erfassen der Antwortstrahlung (F) umfassen,
und
b) eine Abgleicheinrichtung (50) mit einem Referenzelement (52), welches zur Durchführung eines Abgleichs der Untersuchungsvorrichtung (1) von dieser über die Diagnosesonde (10) zu bestrahlen ist,
**dadurch gekennzeichnet,**
**dass** die Abgleicheinrichtung (50) eine Ablage (53) aufweist, über welche die Diagnosesonde (10) in einer definierten Position und/oder Orientierung bezüglich des Referenzelements (52) gehalten ist.

2. Zahnärztliches System nach Anspruch 1,
**dadurch gekennzeichnet,**
**dass** die Abgleicheinrichtung (50) ein Gehäuse (51) aufweist, in dem das Referenzelement (52) gelagert ist,
wobei die Ablage durch eine Ausnehmung (53) in der Gehäusewandung gebildet ist, deren Form an die Kontur der Diagnosesonde (10) angepaßt ist.

3. Zahnärztliches System nach Anspruch 2,
**dadurch gekennzeichnet,**
**dass** die Ausnehmung (53) in der Außenseite der Gehäusewandung gebildet ist, wobei das Gehäuse (51) ferner eine Öffnung (54) aufweist, durch welche sich die Diagnosesonde (10) im abgelegten Zustand erstreckt.

4. Zahnärztliches System nach Anspruch 3,
**dadurch gekennzeichnet,**
**dass** das Referenzelement (52) eine sich zu der Gehäusewandung erstreckende Ausnehmung (52b) aufweist, durch welche sich die Diagnosesonde (10) im abgelegten Zustand erstreckt.

5. Zahnärztliches System nach Anspruch 4,
**dadurch gekennzeichnet,**
**dass** sich die Ausnehmung (52b) von der Mitte des Referenzelements (52) zu der Gehäusewandung erstreckt.

6. Zahnärztliches System nach einem der vorhergehenden Ansprüche,
**dadurch gekennzeichnet,**
**dass** die Diagnosesonde (10) einen aus einem lichtleitenden Material bestehenden länglichen Lichtkeil (11) aufweist, an dessen vorderen Ende eine seitliche Auskopplung der Anregungsstrahlung (A) bzw. Einkopplung der Antwortstrahlung (F) erfolgt,
wobei die Ablage der Abgleicheinrichtung (50) Mittel zur Ausrichtung der Diagnosesonde (10) bezüglich des Referenzelements (52) aufweist.

7. Zahnärztliches System nach einem der Ansprüche 2 bis 5 und Anspruch 6,
**dadurch gekennzeichnet,**
**dass** die Ausnehmung (54) Markierungen aufweist, welche eine korrekte Ausrichtung der Diagnosesonde (10) sicherstellen.

8. Zahnärztliches System nach einem der vorhergehenden Ansprüche,
**dadurch gekennzeichnet,**
**dass** das Referenzelement (52) eine zentrale Vertiefung (52a) aufweist.

9. Zahnärztliches System nach Anspruch 8,
**dadurch gekennzeichnet,**
**dass** die Vertiefung (52a) rotationssymmetrisch ausgestaltet ist.

10. Zahnärztliches System nach Anspruch 8,
**dadurch gekennzeichnet,**
**dass** die Vertiefung (52a) kalottenförmig ausgestaltet ist.

11. Zahnärztliches System nach Anspruch 2,
**gekennzeichnet durch**
einen auf das Gehäuse (51) der Abgleicheinrichtung (50) aufsetzbaren Adapter (60) zur definierten Halterung der Diagnosesonde (10).

12. Zahnärztliches System nach Anspruch 11,
**dadurch gekennzeichnet,**
**dass** der Adapter (60) mit dem Gehäuse (51) der Abgleicheinrichtung (50) verrastbar ist.

13. Zahnärztliches System nach Anspruch 11 oder 12,
**dadurch gekennzeichnet,**
**dass** der Adapter (60) eine horizontal ausgerichtete Grundfläche (61) aufweist, welche eine Öffnung zur Lagerung der Diagnosesonde (10) enthält.

14. Zahnärztliches System nach Anspruch 13,
**dadurch gekennzeichnet,**
**dass** der Adapter (60) derart ausgestaltet ist, dass die Spitze der Diagnosesonde (10) in Anlage gegen die Oberfläche des Referenzelement (52) gelangt.

15. Zahnärztliches System nach einem der Ansprüche 8 bis 10 und Anspruch 14,
**dadurch gekennzeichnet,**
**dass** die Spitze der Diagnosesonde (10) in die Vertiefung (52a) des Referenzelements (52) eintaucht.

16. Abgleicheinrichtung (50) zur Durchführung eines Abgleichs einer Untersuchungsvorrichtung (1), welche
- Mittel zum Erzeugen einer Anregungsstrahlung (A), welche auf einen zu untersuchenden Zahngewebebereich zu lenken ist,
- Erfassungsmitteln (40) und Auswertemitteln zum Erfassen und Bewerten einer von dem bestrahlten Zahngewebebereich als Antwort auf die Bestrahlung entstehenden Antwortstrahlung (F) sowie
- Übertragungsmittel zum Übertragen der Anregungsstrahlung (A) sowie der Antwortstrahlung (F), welche mindestens eine Diagnosesonde (10) zum Ausrichten der Anregungsstrahlung (A) auf den zu untersuchenden Zahngewebebereich sowie zum Erfassen der Antwortstrahlung (F) umfassen,
aufweist,
wobei die Abgleicheinrichtung (50) ein Referenzelement (52) aufweist, welches zur Durchführung des Abgleichs der Untersuchungsvorrichtung (1) von dieser über die Diagnosesonde (10) zu bestrahlen ist,
**dadurch gekennzeichnet,**
**dass** die Abgleicheinrichtung (50) eine Ablage (53) aufweist, über welche die Diagnosesonde (10) in einer definierten Position und/oder Orientierung bezüglich des Referenzelements (52) gehalten ist.

17. Abgleicheinrichtung nach Anspruch 16,
**dadurch gekennzeichnet,**
**dass** diese ein Gehäuse (51) aufweist, in dem das Referenzelement (52) gelagert ist,
wobei die Ablage durch eine Ausnehmung (53) in der Gehäusewandung gebildet ist, deren Form an die Kontur der Diagnosesonde (10) angepaßt ist.

18. Abgleicheinrichtung nach Anspruch 16,
**dadurch gekennzeichnet,**
**dass** die Ausnehmung (53) in der Außenseite der Gehäusewandung gebildet ist, wobei das Gehäuse (51) ferner eine Öffnung (54) aufweist, durch welche sich die Diagnosesonde (10) im abgelegten Zustand erstreckt.

19. Abgleicheinrichtung nach Anspruch 18,
**dadurch gekennzeichnet,**
**dass** das Referenzelement (52) eine sich zu der Gehäusewandung erstreckende Ausnehmung (52b) aufweist, durch welche sich die Diagnosesonde (10) im abgelegten Zustand erstreckt.

20. Abgleicheinrichtung nach Anspruch 19,
**dadurch gekennzeichnet,**
**dass** sich die Ausnehmung (52b) von der Mitte des Referenzelements (52) zu der Gehäusewandung erstreckt.

21. Abgleicheinrichtung nach einem der Ansprüche 16 bis 20,
**dadurch gekennzeichnet,**
**dass** die Diagnosesonde (10) einen aus einem lichtleitenden Material bestehenden länglichen Lichtkeil (11) aufweist, an dessen vorderen Ende eine seitliche Auskopplung der Anregungsstrahlung (A) bzw. Einkopplung der Antwortstrahlung (F) erfolgt,
wobei die Ablage der Abgleicheinrichtung (50) Mittel zur Ausrichtung der Diagnosesonde (10) bezüglich des Referenzelements (52) aufweist.

22. Abgleicheinrichtung nach einem der Ansprüche 17 bis 20 und Anspruch 21,
**dadurch gekennzeichnet,**
**dass** die Ausnehmung (54) Markierungen aufweist, welche eine korrekte Ausrichtung der Diagnosesonde (10) sicherstellen.

23. Abgleicheinrichtung nach einem der Ansprüche 16 bis 22,
**dadurch gekennzeichnet,**
**dass** das Referenzelement (52) eine zentrale Vertiefung (52a) aufweist.

24. Abgleicheinrichtung nach Anspruch 23,
**dadurch gekennzeichnet,**
**dass** die Vertiefung (52a) rotationssymmetrisch ausgestaltet ist.

25. Abgleicheinrichtung System nach Anspruch 24,
**dadurch gekennzeichnet,**
**dass** die Vertiefung (52a) kalottenförmig ausgestaltet ist.

26. Abgleicheinrichtung nach Anspruch 17,
**gekennzeichnet durch**
einen auf das Gehäuse (51) der Abgleicheinrichtung (50) aufsetzbaren Adapter (60) zur definierten Halterung der Diagnosesonde (10).

27. Abgleicheinrichtung nach Anspruch 26,
**dadurch gekennzeichnet,**
**dass** der Adapter (60) mit dem Gehäuse (51) der Abgleicheinrichtung (50) verrastbar ist.

28. Abgleicheinrichtung nach Anspruch 26 oder 27,
**dadurch gekennzeichnet,**
**dass** der Adapter (60) eine horizontal ausgerichtete Grundfläche (61) aufweist, welche eine Öffnung zur Lagerung der Diagnosesonde (10) enthält.

29. Abgleicheinrichtung nach Anspruch 28,
**dadurch gekennzeichnet,**
**dass** der Adapter (60) derart ausgestaltet ist, dass die Spitze der Diagnosesonde (10) in Anlage gegen die Oberfläche des Referenzelement (52) gelangt.

30. Abgleicheinrichtung nach einem der Ansprüche 23 bis 25 und Anspruch 29,
**dadurch gekennzeichnet,**
**dass** die Spitze der Diagnosesonde (10) in die Vertiefung (52a) des Referenzelements (52) eintaucht.
